# EUROPEAN PATENT APPLICATION

(11) **EP 4 765 146 A1**
(43) Date of publication of application: **24.06.2026**
(21) Application number: 24879967.8
(22) Date of filing: 12.09.2024
(51) Int. Cl.: G16H 20/70, G16H 10/60, A61B 5/16, G06V 40/16, G06T 3/4046

(54) **METHOD AND DEVICE FOR MODIFYING FACE OF COUNTERPART, AND DISPLAY DEVICE INCLUDING SAME DEVICE**

(30) Priority: 16.10.2023 KR 20230138075
(71) Applicant: Seoul National University Hospital, Seoul 03080 (KR)
(72) Inventor: HONG, Soonbum, Seoul 03080 (KR); KONG, Hyoun-Joong, Seoul 03080 (KR); KONG, Youngbin, Seoul 03080 (KR)
(74) Representative: BCKIP Part mbB
(86) International application number: PCT/KR2024/013931
(87) International publication number: WO 2025/084628

(57) **Abstract**

A method for deforming a facial image of a counterpart of a user is performed by a counterpart facial morphing apparatus. The method includes: determining state information corresponding to biometric information of the user; deforming the facial image of the counterpart based on the determined state information; and providing the deformed facial image of the counterpart to the user.

## Description

### TECHNICAL FIELD

The present disclosure relates to a method for deforming and providing a counterpart's face, an apparatus for performing the method, and a display device including the apparatus.

For reference, this application claims priority to Korean Patent Application No. 10-2023-0138075, filed October 16, 2023. The entire contents of that application, which serves as the basis for this priority claim, are incorporated herein by reference.

### BACKGROUND ART

With the development of technology in the image processing field, facial morphing technology, such as changing the facial expression in a facial image, is implemented in various forms. For example, a 3D model of an avatar may be processed to have various facial expression changes.

Meanwhile, according to expert opinions in psychiatry, people lacking confidence in interpersonal relationships and having high levels of anxiety tend to amplify their anxiety by interpreting others' facial expressions negatively. That is, factors maintaining anxiety disorders arise by amplifying objectively unfounded or unrealistic erroneous thoughts. Therefore, treatment is needed to ultimately raise the threshold for anxiety by overcoming maintaining factors such as 'anxiety about anxiety itself', rather than factors causing anxiety disorders. Behavioral therapy for this treatment aims to reduce fundamental anxiety while correcting and eliminating abnormal behaviors amplifying or maintaining anxiety. One of the common abnormal behaviors exhibited in anxiety disorders is 'avoidance behavior', and this avoidance behavior helps to relieve some anxiety at first, but later exacerbates the degree of anxiety. Therefore, conversely, a therapeutic effect of reducing abnormal behavior or anxiety may be obtained by having a person confront and attempt exposure to a feared situation or place; this is called 'exposure therapy' and is the most representative technique among behavioral therapies.

### (Prior Art Document)

(Patent Document 1) Korean Patent No. 10-2507654 (Published February 28, 2023)

### DETAILED DESCRIPTION OF INVENTION

### TECHNICAL PROBLEMS

The present disclosure provides a method and apparatus for deforming a counterpart's face, deforming and providing a counterpart's facial image based on biometric information of a user, to enable a facial morphing technology, such as changing the facial expression of a facial image, to be applied to exposure therapy in the field of psychiatry.

Furthermore, the present disclosure provides a display device capable of reproducing a counterpart's facial image deformed based on biometric information of a user.

The problems to be solved by the present disclosure are not limited to those described above, and other unmentioned problems to be solved will be clearly understood by those of ordinary skill in the art to which the present disclosure pertains from the following description.

### TECHNICAL SOLUTION

In accordance with a first aspect of the present disclosure, there is provided a method for deforming a facial image of a counterpart of a user, performed by a facial morphing apparatus, the method including: determining state information corresponding to biometric information of the user; deforming a facial image of the counterpart based on the determined state information; and providing the deformed facial image of the counterpart to the user.

In accordance with a second aspect of the present disclosure, there is provided a counterpart face morphing apparatus including: a memory in which a face deformation program is stored; and a processor programmed to load the face deformation program from the memory and execute the face deformation program, wherein the processor is programmed to determine state information corresponding to biometric information of a user, deform a facial image of a counterpart of the user based on the determined state information, and provide the deformed facial image of the counterpart to the user.

In accordance with a third aspect of the present disclosure, there is provided a non-transitory computer-readable recording medium on which a computer program is stored, wherein the computer program, when executed by a processor, includes instructions for causing the processor to perform a method for deforming a facial image of a counterpart of a user, the method including: determining state information corresponding to biometric information of the user; deforming the facial image of the counterpart based on the determined state information; and providing the deformed facial image of the counterpart to the user.

In accordance with a fourth aspect of the present disclosure, there is provided a display device including the counterpart face morphing apparatus.

### EFFECT OF INVENTION

According to an embodiment, by deforming and providing a counterpart's facial image based on user biometric information, such as a biometric indicator value measured through a biosensor or a user's facial image, facial morphing technology, such as changing the facial expression of a facial image, may be applied to exposure therapy in the field of psychiatry. In one embodiment, the counterpart's facial image deformed based on the user's biometric information may be provided through a display device.

Furthermore, by reflecting the result of identifying a change in the biometric information of the user after providing the deformed counterpart's facial image when subsequently deforming the counterpart's facial image, a service that immediately reflects the progress of exposure therapy may be provided. When providing a service to a user in a state of tension, a more flexible and personalized experience may be provided through optimization by adjusting a threshold value, signifying a reference limit of the tension level manageable by the user, and a therapy intensity.

Furthermore, because various data (e.g., tension level, threshold value, therapy intensity) generated during the service process may also be accumulated, a clinician may monitor the progress and effects of the therapy. Through continuous monitoring, phenomena such as symptom aggravation are identified early and appropriate support is provided to the user, ultimately having an effect of preventing or minimizing the impact of disruptions in the treatment process.

### BRIEF DESCRIPTION OF THE DRAWINGS

FIG. 1 is a block diagram of a counterpart image reproduction system including a counterpart face morphing apparatus according to an embodiment of the present disclosure.
FIG. 2 is a block diagram illustrating functions of the face deformation program shown in FIG. 1.
FIGS. 3 to 5 are flowcharts illustrating a method for deforming a counterpart's face according to an embodiment of the present disclosure.

### DETAILED DESCRIPTION OF THE INVENTION

The advantages and features of the present disclosure, and the methods for achieving them, will become clear by referring to the embodiments described below with the accompanying drawings. However, the present disclosure is not limited to the embodiments disclosed below but may be implemented in various different forms, and these embodiments are provided only to make the disclosure of the present disclosure complete and to fully inform the scope of the invention to those skilled in the art to which the present disclosure pertains, and the present disclosure is only defined by the scope of the claims.

The terms used in this specification will be briefly described, and the present disclosure will be described in detail.

The terms used in the present disclosure have been selected from general terms widely used at present, taking into account their functions in the present disclosure, but they may vary depending on the intention of a person skilled in the art, legal precedents, or the emergence of new technologies. Furthermore, in specific cases, there are terms arbitrarily selected by the applicant, and in such cases, their meanings will be described in detail in the corresponding description section of the disclosure. Therefore, the terms used in the present disclosure should be defined based on the meaning they have and the overall content of the present disclosure, not just their simple names.

Throughout the specification, when a part is said to 'include' a certain component, this means that the part may include other components, rather than excluding them, unless there is a specific statement to the contrary.

Furthermore, the term 'unit' used in the specification refers to a software or hardware component such as an FPGA (field programmable gate array) or ASIC (application-specific integrated circuit), and the 'unit' performs certain roles. However, 'unit' is not limited to software or hardware. A 'unit' may reside in an addressable storage medium and may be executed by one or more processors. Therefore, as an example, a 'unit' includes components such as software components, object-oriented software components, class components, and task components, as well as processes, functions, attributes, procedures, subroutines, segments of program code, drivers, firmware, microcode, circuits, data, databases, data structures, tables, arrays, and variables. The functions provided in the components and 'units' may be combined into a smaller number of components and 'units' or further separated into additional components and 'units'.

Hereinafter, embodiments of the present disclosure will be described in detail with reference to the accompanying drawings so that a person of ordinary skill in the art to which the present disclosure pertains may easily implement them. In the drawings, portions irrelevant to the description are omitted to clearly describe the present disclosure.

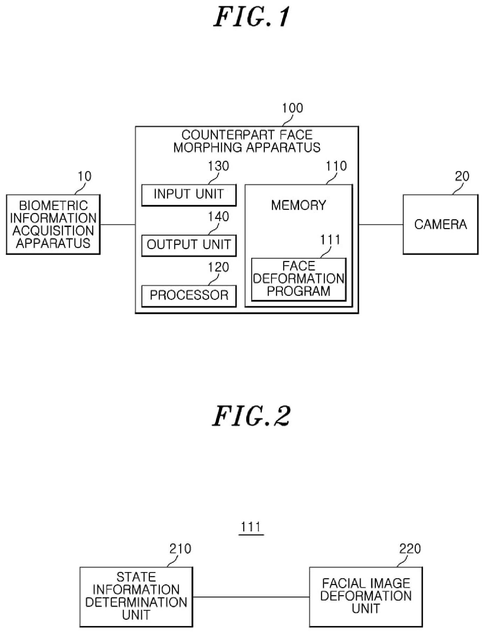

FIG. 1 is a block diagram of a counterpart image reproduction system including a counterpart face morphing apparatus according to an embodiment of the present disclosure.

Referring to FIG. 1, a counterpart image reproduction system according to an embodiment may include a biometric information acquisition apparatus 10, a camera 20, and a counterpart face morphing apparatus 100. The counterpart face morphing apparatus 100 includes a memory 110 in which a face deformation program 111 is stored and a processor 120, and may further include an input unit 130 and/or an output unit 140.

The biometric information acquisition apparatus 10 may acquire a biometric indicator value measured through at least one biosensor as biometric information and provide it to the counterpart face morphing apparatus 100. In one embodiment, the biometric information acquisition apparatus 10 may sense a biometric indicator value of a user using at least one sensor from among an ECG (electrocardiogram) sensor, a PPG (photoplethysmography) sensor, an EEG (electroencephalogram) sensor, an EDA (electrodermal activity) sensor, an EMG (electromyography) sensor, a respiration sensor, an EOG (electro-oculography) sensor, and a VOG (videooculography) sensor, and provide it to the counterpart face morphing apparatus 100. Alternatively, the biometric information acquisition apparatus 10 may acquire a user's facial image as biometric information and provide it to the counterpart face morphing apparatus 100.

The camera 20 captures a counterpart's image and provides the captured image to the counterpart face morphing apparatus 100. For example, the camera 20 may include an RGB camera, an infrared camera, a depth camera, etc., but is not limited thereto.

The counterpart face morphing apparatus 100 may perform the method for deforming a counterpart's face according to an embodiment when the processor 120 executes the face deformation program 111 in the memory 110. Accordingly, the counterpart face morphing apparatus 100 determines state information corresponding to the biometric information of the user provided from the biometric information acquisition apparatus 10, deform the counterpart's facial image according to the determined state information, and provides the deformed counterpart's facial image.

The memory 110 of the counterpart face morphing apparatus 100 may store the face deformation program 111 including instructions executable by the processor 120, and may further store various information necessary for the execution of the face deformation program 111. For example, the face deformation program 111 may refer to software including instructions programmed to cause the counterpart face morphing apparatus 100 to deform the counterpart's facial image according to the state information of the user for a counterpart image provided by the camera 20 or pre-stored in the memory 110.

The processor 120 of the counterpart face morphing apparatus 100 may load the face deformation program 111 from the memory 110 to execute the face deformation program 111, and may load information necessary for the execution of the face deformation program 111 together.

The processor 120, by executing the instructions of the face deformation program 111, may perform the method for deforming a counterpart's face according to an embodiment of the present disclosure. As such, by performing the method for deforming a counterpart's face, the processor 120 may determine the state information of the user based on the level of a biometric indicator value provided by the biometric information acquisition apparatus 10. Alternatively, the processor 120 may determine the emotion represented in the user's facial image provided by the biometric information acquisition apparatus 10 as the state information of the user. Then, the processor 120 deforms the counterpart's facial image according to the determined user state information, and may provide the deformed counterpart's facial image.

FIG. 1 illustrates an embodiment in which the biometric information acquisition apparatus 10 and the camera 20 are implemented separately from the counterpart face morphing apparatus 100 and interwork with it, and this embodiment has been described above, but this is merely an embodiment. In one embodiment, the counterpart face morphing apparatus 100 and the biometric information acquisition apparatus 10 and/or the camera 20 may be integrated into a single unit.

The functions and/or operations of the face deformation program 111 illustrated in FIG. 1 will be described in detail with reference to FIG. 2.

Referring to FIG. 2, the face deformation program 111 may include a state information determination unit 210 and a facial image deformation unit 220.

The face deformation program 111 is functionally divided into the state information determination unit 210 and the facial image deformation unit 220, as illustrated in FIG. 2, to aid understanding of the face deformation program 111, and is not limited to such a functional division. According to embodiments, the respective functions by the state information determination unit 210 and the facial image deformation unit 220 may be merged and may be implemented as a series of instructions included in a single program.

The state information determination unit 210 may determine the state information of the user based on the level of a biometric indicator value provided by the biometric information acquisition apparatus 10. For example, the state information determination unit 210 may determine the state information of the user based on at least one biometric indicator value from among an ECG sensing value, a PPG sensing value, an EEG sensing value, an EDA sensing value, an EMG sensing value, a respiration sensing value, an EOG sensing value, and a VOG sensing value. For instance, the state information determination unit 210 may determine a tension level, a stress index, etc., as user state information according to the levels of a plurality of biometric indicator values. As such, calculating a tension level, a stress index, or the like based on various biometric indicator values corresponds to a known technical concept, a detailed description thereof is omitted. Alternatively, the state information determination unit 210 may determine the emotion represented in the user's facial image as the state information of the user. For instance, the state information determination unit 210 may identify a facial expression element (e.g., eyebrows, eyes, crow's feet, nose, smile lines) from the user's facial image and determine the emotion (e.g., anger, surprise, disgust, sadness, fear, joy, contempt) corresponding to the identified facial expression element and its intensity as user state information. Alternatively, the state information determination unit 210 may represent the emotion corresponding to the facial expression element identified from the user's facial image as a concept of positive/negative based on two axes and determine it as user state information. As such, determining an emotion and its intensity from facial expression elements of a facial image or representing an emotion as a concept of positive/negative based on two axes corresponds to a known technical concept, a detailed description thereof is omitted.

The facial image deformation unit 220 deforms the counterpart's facial image according to the user state information determined by the state information determination unit 210. Here, the facial image deformation unit 220 may deform the counterpart's facial image by reflecting at least one of a predetermined threshold value and a medical finding (e.g., clinician's diagnosis, prescription, treatment plan) corresponding to the user. The threshold value may signify a reference limit of the tension level manageable by the user. In one embodiment, deforming a facial image may be changing an emotion (e.g., anger, surprise, disgust, sadness, fear, joy, contempt) shown by facial expression elements (e.g., eyebrows, eyes, crow's feet, nose, smile lines) of the facial image or adjusting its intensity. Alternatively, changing a facial image may be changing the concept of positive/negative (positive direction and negative direction) based on two axes for an emotion. For instance, a counterpart facial image deformation rule corresponding to user state information, in which a medical finding is reflected, may be pre-stored in the memory 110 and/or an internal memory of the processor 120, or may be input in real time through the input unit 130.

When deforming the counterpart's facial image, the facial image deformation unit 220 may acquire a facial feature point from the counterpart's facial image and perform image processing that changes the position of the previously acquired facial feature point with respect to the counterpart's facial image according to the state information determined by the state information determination unit 210.

Alternatively, when changing the counterpart's facial image, the facial image deformation unit 220 may set a deformation target according to the state information determined by the state information determination unit 210, and input the counterpart's facial image into a pre-trained artificial neural network model to obtain the counterpart's facial image deformed by the previously set deformation target as the output of the artificial neural network model.

Furthermore, after providing the deformed counterpart's facial image, the facial image deformation unit 220 may identify a change in the biometric information acquired by the biometric information acquisition apparatus 10, and reflect the identified change in the biometric information when subsequently deforming the counterpart's facial image. For example, information about a change in the biometric information acquired by the biometric information acquisition apparatus 10 after providing the deformed counterpart's facial image may be provided to the outside through the output unit 140. Information about such a change in the biometric information may be analyzed by a clinician or the like, and may be reflected through a treatment plan by the clinician or the like when the facial image deformation unit 220 subsequently deforms the counterpart's facial image.

Referring to FIG. 1 again, the input unit 130 may receive various information necessary for the processor 120 to perform the method for deforming a counterpart's face. Such various information may be input in real time, and when input in advance, the various information may be stored in the memory 110. In one embodiment, the input unit 130 may receive a biometric indicator value measured by the biometric information acquisition apparatus 10 and provide it to the processor 120, and may receive a counterpart's image captured by the camera 20 and provide it to the processor 120.

The output unit 140 may output various processing data generated as the processor 120 performs the method for deforming a counterpart's face by the face deformation program 111. For example, the output unit 140 may include a data interface capable of outputting various processing data to external peripheral devices, a communication module capable of transmitting various processing data through a communication channel, a reproduction device capable of reproducing various processing data so that they may be visually or audibly perceived externally, and so on. In one embodiment, the output unit 140 may reproduce the counterpart's facial image deformed by the processor 120 for the user to see or transmit it to the outside through a communication module. In one embodiment, when the output unit 140 reproduces a counterpart's image including the deformed counterpart's facial image on a screen according to the control of the processor 120, the counterpart face deformation apparatus 100 may be a display device. In one embodiment, the processor 120 included in the display device may provide the deformed counterpart's facial image as a virtual reality (VR) image, an augmented reality (AR) image, a mixed reality (MR) image, an extended reality (XR) image, etc. through the output unit 140. As such, providing a counterpart's facial image in various forms of images is a known technical concept, so a detailed description thereof is omitted. Furthermore, the display device may be implemented not only as a wearable display device such as a head mounted display (HMD), smart glasses, or a headset, but also as a general flat panel 2D/3D/4D display device.

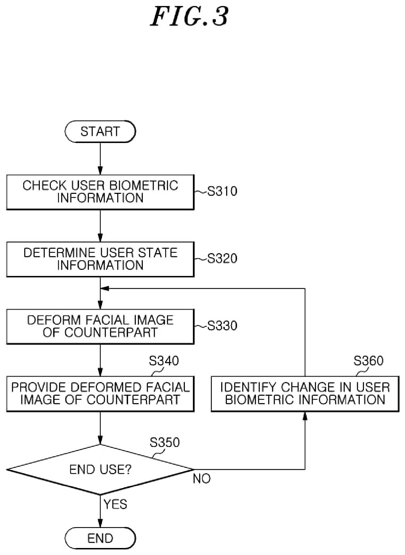

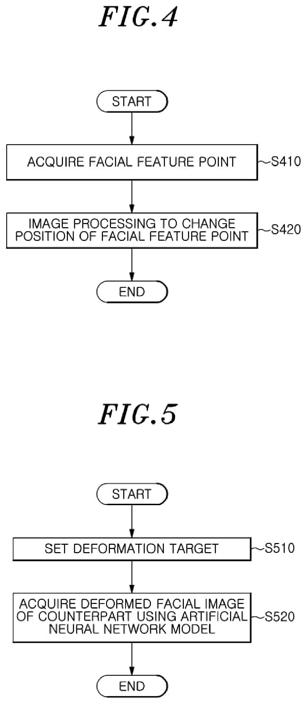

FIGS. 3 to 5 are flowcharts illustrating a method for deforming a counterpart's face according to an embodiment of the present disclosure.

Hereinafter, the process in which a counterpart's facial image is reproduced and shown to a user by a counterpart image reproduction system including a counterpart face morphing apparatus 100 according to an embodiment will be described in detail with reference to FIGS. 1 to 5.

First, the processor 120 of the counterpart face morphing apparatus 100 may load the face deformation program 111 from the memory 110 and load information necessary for the execution of the face deformation program 111 together, and by executing the instructions of the loaded face deformation program 111, the processor 120 may perform the method for deforming a counterpart's face according to an embodiment of the present disclosure.

The biometric information acquisition apparatus 10 may acquire a biometric indicator value measured through at least one biosensor as biometric information and provide it to the counterpart face morphing apparatus 100. For example, the biometric information acquisition apparatus 10 may acquire at least one of an ECG sensing value, a PPG sensing value, an EEG sensing value, an EDA sensing value, an EMG sensing value, a respiration sensing value, an EOG sensing value, and a VOG sensing value as biometric information and provide it to the counterpart face morphing apparatus 100. Alternatively, the biometric information acquisition apparatus 10 may acquire a user's facial image as biometric information and provide it to the counterpart face morphing apparatus 100. Then, the camera 20 captures a counterpart's image and provides it to the counterpart face morphing apparatus 100. As such, the biometric information provided to the biometric information acquisition apparatus 10 and the counterpart image provided by the camera 20 are provided to the processor 120 through the input unit 130 (S310).

Then, the state information determination unit 210 of the face deformation program 111 executed by the processor 120 of the counterpart face morphing apparatus 100 may determine the state information of the user based on the level of the biometric indicator value provided by the biometric information acquisition apparatus 10. For example, the state information determination unit 210 may determine a tension level, a stress index, etc., as user state information according to the levels of a plurality of biometric indicator values. As such, calculating a tension level, a stress index, or the like based on various biometric indicator values corresponds to a known technical concept, a detailed description thereof is omitted. Alternatively, the state information determination unit 210 may determine the emotion represented in the user's facial image as the state information of the user. For instance, the state information determination unit 210 may identify a facial expression element (e.g., eyebrows, eyes, crow's feet, nose, smile lines) from the user's facial image and determine the emotion (e.g., anger, surprise, disgust, sadness, fear, joy, contempt) corresponding to the identified facial expression element and its intensity as user state information. Alternatively, the state information determination unit 210 may represent the emotion corresponding to the facial expression element identified from the user's facial image as a concept of positive/negative (positive direction and negative direction) based on two axes and determine it as user state information. As such, determining an emotion and its intensity from facial expression elements of a facial image or representing an emotion as a concept of positive/negative based on two axes corresponds to a known technical concept, a detailed description thereof is omitted (S320).

Next, the facial image deformation unit 220 of the face deformation program 111 executed by the processor 120 of the counterpart face morphing apparatus 100 deforms the counterpart's facial image according to the user state information determined by the state information determination unit 210. Here, the facial image deformation unit 220 may deform the counterpart's facial image by applying at least one of a predetermined threshold value and a medical finding (e.g., clinician's diagnosis, prescription, treatment plan) corresponding to the user. The threshold value may signify a reference limit of the tension level manageable by the user. For example, deforming a facial image may be changing an emotion (e.g., anger, surprise, disgust, sadness, fear, joy, contempt) shown by facial expression elements (e.g., eyebrows, eyes, crow's feet, nose, smile lines) of the facial image or adjusting its intensity. Alternatively, changing a facial image may be changing the concept of positive/negative (positive direction and negative direction) based on two axes for an emotion. For instance, a counterpart facial image deformation rule corresponding to user state information, reflecting a medical finding, may be pre-stored in the memory 110 and/or an internal memory of the processor 120, or may be input in real time through the input unit 130. Furthermore, the adjustment of the threshold value and/or intensity may reflect an optimized result through a deep learning-based algorithm (S330).

Here, when deforming the counterpart's facial image, the facial image deformation unit 220 may acquire a facial feature point from the counterpart's facial image (S410), and perform image processing that changes the position of the previously acquired facial feature point with respect to the counterpart's facial image according to the state information determined by the state information determination unit 210 (S420).

Alternatively, when changing the counterpart's facial image, the facial image deformation unit 220 may set a deformation target according to the state information determined by the state information determination unit 210 (S510), and input the counterpart's facial image into a pre-trained artificial neural network model to obtain the counterpart's facial image deformed by the previously set deformation target as the output of the artificial neural network model (S520). In one embodiment, when using a generative adversarial network (GAN) as the artificial neural network model, a preprocessing process of extracting facial feature points from the counterpart's facial image may be performed, the preprocessing result may be input into the pre-trained generative adversarial network, and the deformed counterpart's facial image may be obtained as the output of the generative adversarial network.

The counterpart's facial image thus deformed by the processor 120 may be reproduced through the output unit 140 for the user to see or may be transmitted to the outside through a communication module. In one embodiment, a display device including the counterpart face morphing apparatus 100 may provide a counterpart image including the deformed counterpart's facial image as a virtual reality (VR) image, an augmented reality (AR) image, a mixed reality (MR) image, an extended reality (XR) image, etc. according to the control of the processor 120. As such, providing a counterpart's facial image in various forms of images is a known technical concept, so a detailed description thereof is omitted (S340).

Meanwhile, after providing the deformed counterpart's facial image through the output unit 140, the counterpart face morphing apparatus 100 may identify a change in the biometric information acquired by the biometric information acquisition apparatus 10, and reflect the identified change in the biometric information when subsequently deforming the counterpart's facial image. In one embodiment, information about a change in the biometric information acquired by the biometric information acquisition apparatus 10 after providing the deformed counterpart's facial image may be provided to the outside through the output unit 140. Information about such a change in the biometric information may be analyzed by a clinician or the like, and may be reflected through a treatment plan by the clinician or the like when the facial image deformation unit 220 subsequently deforms the counterpart's facial image. For example, a medical finding after providing the deformed counterpart's facial image may be reflected in a counterpart facial image deformation rule that changes an emotion (e.g., anger, surprise, disgust, sadness, fear, joy, contempt) shown by facial expression elements (e.g., eyebrows, eyes, crow's feet, nose, smile lines) of the facial image or adjusts its intensity. Alternatively, a medical finding after providing the deformed counterpart's facial image may be reflected in a counterpart facial image deformation rule that represents an emotion corresponding to the facial expression elements of the facial image as a concept of positive/negative (positive direction and negative direction) based on two axes (S350, S360).

As described so far, according to an embodiment of the present disclosure, by deforming and providing a counterpart's facial image based on user biometric information such as a biometric indicator value measured through a biosensor or a user's facial image, it is possible to apply facial morphing technology, such as changing the facial expression of a facial image, to exposure therapy in the field of psychiatry. In one embodiment, the counterpart's facial image deformed based on the biometric information of the user may be provided through a display device.

Furthermore, by reflecting the result of identifying a change in the biometric information of the user after providing the deformed counterpart's facial image when subsequently deforming the counterpart's facial image, a service that immediately reflects the progress of exposure therapy may be provided. When providing a service to a user in a state of tension, a more flexible and personalized experience may be provided through optimization by adjusting a threshold value, signifying a reference limit of the tension level manageable by the user, and a therapy intensity.

Furthermore, because various data (e.g., tension level, threshold value, therapy intensity) generated during the service process may also be accumulated, a clinician may monitor the progress and effects of the therapy. Through continuous monitoring, phenomena such as symptom aggravation are identified early and appropriate support is provided to the user, ultimately having an effect of preventing or minimizing the impact of disruptions in the treatment process.

Meanwhile, a computer program may be implemented to include instructions for causing a processor to perform each operation included in the method for deforming a counterpart's face according to the aforementioned embodiment.

Furthermore, a computer program including instructions for causing a processor to perform each operation included in the method for deforming a counterpart's face according to the aforementioned embodiment may be recorded on a computer-readable recording medium.

Combinations of operations in each of the flowcharts attached to the present disclosure may also be performed by computer program instructions. These computer program instructions may be loaded into a processor of a general-purpose computer, a special-purpose computer, or other programmable data processing equipment, so that the instructions performed through the processor of the computer or other programmable data processing equipment generate means for performing the functions described in each operation of the flowcharts. These computer program instructions may also be stored in a computer-usable or computer-readable recording medium directing a computer or other programmable data processing equipment to function in a particular manner, so that the instructions stored in the computer-usable or computer-readable recording medium may also produce an article of manufacture embodying instruction means for performing the functions described in each operation of the flowcharts. The computer program instructions may also be loaded onto a computer or other programmable data processing equipment, so that a series of operations are performed on the computer or other programmable data processing equipment to generate a computer-implemented process, so that the instructions executing on the computer or other programmable data processing equipment may also provide operations for executing the functions described in each operation of the flowcharts.

Furthermore, each operation may represent a module, segment, or portion of code, which includes one or more executable instructions for executing the specified logical function(s). It should also be noted that in some alternative embodiments, the functions mentioned in the operations may occur out of order. For example, two operations shown in succession may in fact be performed substantially concurrently or the operations may sometimes be performed in reverse order, depending on the functionality involved.

The foregoing description is merely an exemplary illustration of the technical idea of the present disclosure, and various modifications and variations will be possible for a person of ordinary skill in the art to which the present disclosure pertains without departing from the essential quality of the present disclosure. Therefore, the embodiments disclosed in the present disclosure are not intended to limit the technical idea of the present disclosure but to describe it, and the scope of the technical idea of the present disclosure is not limited by these embodiments. The scope of protection of the present disclosure should be construed by the claims below, and all technical ideas within the equivalent scope should be construed as being included in the scope of rights of the present disclosure.

## Claims

1. A method for deforming a facial image of a counterpart of a user, performed by a facial morphing apparatus, the method comprising:
determining state information corresponding to biometric information of the user;
deforming the facial image of the counterpart based on the determined state information; and
providing the deformed facial image of the counterpart.

2. The method of claim 1, wherein the determining includes:
acquiring a biometric indicator value measured through at least one biosensor as the biometric information; and
determining the state information based on a level of the biometric indicator value.

3. The method of claim 1, wherein the determining includes:
acquiring a facial image of the user as the biometric information; and
determining an emotion represented in the facial image as the state information.

4. The method of claim 1, wherein the deforming includes:
acquiring a facial feature point from the facial image of the counterpart; and
performing image processing to change a position of the acquired facial feature point in the facial image of the counterpart based on the determined state information.

5. The method of claim 1, wherein the deforming includes:
setting a deformation target based on the determined state information; and
inputting the facial image of the counterpart into a pre-trained artificial neural network model to acquire the deformed facial image of the counterpart based on the deformation target as an output of the artificial neural network model.

6. The method of claim 1, further comprising:
identifying a change in the biometric information after providing the deformed facial image of the counterpart; and
deforming the facial image of the counterpart by applying the identified change in the biometric information.

7. The method of claim 6, wherein the deforming includes deforming the facial image of the counterpart by applying at least one of a predetermined threshold value and a medical finding, each corresponding to the user.

8. A counterpart face morphing apparatus comprising:
a memory in which a face deformation program is stored; and
a processor programmed to load the face deformation program from the memory and execute the face deformation program,
wherein the processor is programmed to determine state information corresponding to biometric information of a user, deform a facial image of a counterpart of the user based on the determined state information, and provide the deformed facial image of the counterpart.

9. The counterpart face morphing apparatus of claim 8, wherein the processor is programmed to acquire a biometric indicator value measured through at least one biosensor as the biometric information, and then determine the state information based on a level of the biometric indicator value.

10. The counterpart face morphing apparatus of claim 8, wherein the processor is programmed to acquire a facial image of the user as the biometric information, and then determine an emotion represented in the facial image as the state information.

11. The counterpart face morphing apparatus of claim 8, wherein the processor is programmed to, when deforming the facial image of the counterpart, acquire a facial feature point from the facial image of the counterpart, and perform image processing to change a position of the acquired facial feature point in the facial image of the counterpart based on the determined state information.

12. The counterpart face morphing apparatus of claim 8, wherein the processor is programmed to, when deforming the facial image of the counterpart, set a deformation target based on the determined state information, and input the facial image of the counterpart into a pre-trained artificial neural network model to acquire a deformed facial image of the counterpart based on the deformation target as an output of the artificial neural network model.

13. The counterpart face morphing apparatus of claim 8, wherein the processor is programmed to identify a change in the biometric information after providing the deformed facial image of the counterpart, and deform the facial image of the counterpart by applying the identified change in the biometric information.

14. The counterpart face morphing apparatus of claim 13, wherein the processor is programmed to deform the facial image of the counterpart by applying at least one of a predetermined threshold value and a medical finding, each corresponding to the user.

15. A non-transitory computer-readable recording medium on which a computer program is stored, wherein the computer program, when executed by a processor, comprises instructions for causing the processor to perform a method for deforming a facial image of a counterpart of a user, the method comprising:
determining state information corresponding to biometric information of the user;
deforming the facial image of the counterpart based on the determined state information; and
providing the deformed facial image of the counterpart to the user.
